(19) Europäisches Patentamt
European Patent Office
Office européen des brevets

(11) Veröffentlichungsnummer: **0 313 712 B1**

(12) **EUROPÄISCHE PATENTSCHRIFT**

(45) Veröffentlichungstag der Patentschrift: **29.07.92**

(51) Int. Cl.[5]: **A61K 49/02**

(21) Anmeldenummer: **87810624.4**

(22) Anmeldetag: **30.10.87**

# (54) Stabile radiodiagnostische Präparate und ihre Herstellung.

(43) Veröffentlichungstag der Anmeldung:
**03.05.89 Patentblatt 89/18**

(45) Bekanntmachung des Hinweises auf die Patenterteilung:
**29.07.92 Patentblatt 92/31**

(84) Benannte Vertragsstaaten:
**BE CH DE FR GB IT LI LU NL**

(56) Entgegenhaltungen:
**EP-A- 0 004 684**

**CHEMICAL ABSTRACTS, Band 92, Nr. 7, 18. Februar 1980, Seiten 539-540, Zusammenfassung Nr. 57067f, Columbus, Ohio, US; G. KAJIMOTO et al.: "Studies on the antioxidative activities of some amino acid metabolites", EIYO TO SHOKURYO 1979, 32(1), 41-6**

**Inncl. Med. I 31-35 (1976)**

(73) Patentinhaber: **SORIN BIOMEDICA S.p.A.**
**Strada per Crescentino**
**I-13040 Saluggia (Vercelli)(IT)**

(72) Erfinder: **Chia, Han-Lie, Dr.**
**Mischelistrasse 35**
**CH-4153 Reinach(CH)**
Erfinder: **Popescu, Horia, Dr.**
**Mariasteinstrasse 8**
**CH-4054 Basel(CH)**
Erfinder: **Boehmer-Ackermann, Gabrielle**
**Sonnenhofring 30**
**Reinach(CH)**

(74) Vertreter: **Rambelli, Paolo et al**
**Jacobacci-Casetta & Perani Via Alfieri, 17**
**P.O. Box 321**
**I-10121 Torino Centro(IT)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind stabile, gebrauchsfertige, mit dem Radionuklid Tc-99m markierte Präparate zur Anwendung als Radiodiagnostika, ein Verfahren zur Herstellung derselben und Mittel zur Ausführung des Verfahrens.

Die Skelettszintigraphie mit $^{99m}$Tc-Methylendiphosphonat ($^{99m}$Tc-MDP) stellt heute eine unentbehrliche Methode zur Diagnose pathologischer Knochenprozesse dar. Ihr Platz in der Nuklearmedizin hat während der letzten Jahre ständig an Bedeutung zugenommen. In der Tat wird diese Methode wegen ihrer Empfindlichkeit im Nachweis von Störungen des Knochenstoffwechsels für die Frühdiagnose von Knochenmetastasen in der postoperativen Versorgung bestimmter Krebsarten in programmierten Zeitabständen eingesetzt.

Da täglich mehrere Patienten den nuklearmedizinischen Laboratorien zur Skelettszintigraphie zugewiesen werden, andererseits die optimale Abstandszeit zwischen der Verabreichung von $^{99m}$Tc-MDP oder ähnlichen Präparaten und der Szintigraphie 2 bis 3 Stunden beträgt, wäre es von Vorteil, über ein Radiopharmakon zu verfügen, welches morgens früh mit einer hohen Radioaktivitätsmenge von $^{99m}$Tc-Pertechnetat markiert werden kann, und aus welchem über den ganzen Tag soviele Einzeldosen entnommen werden können, wie Patienten zur Szintigraphie zugewiesen werden.

Ferner sind, vor allem in den USA, Grossbritannien und Holland, sogenannte "Radiopharmacies" entstanden. Ueblicherweise führen sie selbst keine nuklearmedizinischen Untersuchungen durch, sondern beliefern täglich kleinere Kliniken, nuklearmedizinische Laboratorien und Privatpraxen mit injektionsfertigen Einzeldosen von Präparaten. Für diese Zentralstellen ist es wichtig, stabile Radiopharmaka zur Verfügung zu haben; sie können dann einen erweiterten Kundenkreis in grösserer Entfernung bedienen.

Nun sind die mit Tc-99m markierten Injektionslösungen, im Gegensatz zu den Lyophilisaten aus organspezifischem Trägerstoff und Reduktionsmittel (z.B. Zinn(II)salz), bekanntlich nur beschränkt haltbar. Schon wenige Stunden nach der Markierung, insbesondere bei hoher Tc-99m-Aktivitätsmenge, lässt sich in dem Injektionslösung die Anwesenheit von freiem Pertechnetat feststellen; sie vermindert die Qualität der Präparate. Freies Pertechnetat wird nämlich im Magen und in der Schilddrüse angereichert und verursacht dadurch eine unnötige Strahlenbelastung. Ferner stört es die klare Darstellung der Zielorgane oder -gewebe durch einen Strahlungsuntergrund in den Weichteilen.

Die Rückbildung von freiem Pertechnetat aus den Technetiumionen geht auf die oxidierende Wirkung von in der Lösung gegebenenfalls vorhandenem Restsauerstoff, vor allem aber auf jene von verschiedenen radiolytischen Produkten, insbesondere von Radikalen und Peroxiden zurück; als Folge der Radiolyse entstehen letztere in wässriger Lösung fortlaufend. Ihrerseits wird die Radiolyse durch die Anwesenheit von Restsauerstoff und schon durch Spuren von Schwermetallionen katalysiert.

Eigene Untersuchungen von Eluaten aus dem Tc-99m-Generator haben gezeigt, dass der Gehalt an Peroxiden in Eluaten, welche bis zur Anwendung für die Markierungen von Radiopharmaka normalerweise in der Praxis stehengelassen werden, im Lauf der Zeit zunimmt. Die Tabelle 1 zeigt, dass die Peroxide schneller gebildet werden, je höher die Anfangsaktivität von Tc-99m in den Eluaten ist (Peroxid-Bestimmung semiquantitativ mittels Merckoquant-Teststäbchen, Merck Art. Nr. 10011). Die Nachweisgrenze der Methode beträgt 1 ppm.

Tabelle 1

| Peroxidgehalt (in ppm) von Tc-99m-Eluaten aus Tc-99m-Generatoren | | | | | |
|---|---|---|---|---|---|
| Standzeit nach Elution | 57,2 GBq (1546 mCi) | 41,5 GBq (1122 mCi) | 32,9 GBq (889 mCi) | 27,9 GBq (755 mCi) | 12,2 GBq (330 mCi) |
| 0-1 Std. | 0 | 0 | 0 | 0 | 0 |
| 3 Std. | 1 | 1 | 1 | - | - |
| 5 Std. | 3 | 1-3 | 1-3 | 1-2 | 0 |
| 8 Std. | -[1)] | 3 | 3 | - | - |
| 26 Std. | - | - | - | 0-1 | 0 |
| 48 Std. | - | - | - | 0 | 0 |

1): nicht bestimmt

Zur Stabilisierung der $^{99m}$Tc-markierten Injektionslösungen hat man u.a. die Verwendung verschiedener chemischer Substanzen und vor allem der Komplexbildner vorgeschlagen. In dieser Hinsicht sind z.B. daS

Cystein [J. Nucl. Med. 22 (1981), 645], das Hydrochinon (US-A-4 229 427), der Gentisylalkohol (US-A-4 232 000) und viele andere komplexbildende Verbindungen (US-A-4 027 005) untersucht worden.

Besonders empfohlen wurde die Verwendung einerseits der Reduktinsäuren, vor allem der Ascorbinsäure, ihrer Salze und Ester (DE-A-2 618 337, DE-A-2 619 382, DE-A-2 660 424; US-A-075 314, US-A-4 440 738, US-A-4 504 462; CA-A-1 114 291), andererseits der Hydroxy- und Aminobenzoesäuren, insbesondere der 2,5-Dihydroxybenzoesäure oder Gentisinsäure und ihrer Salze (US-A-4 233 284, US-A-4 451 451, US-A-4 504 463; DE-A-3 331 159; EP-A-4684; WO 85/3231). Eine effektive Verwendung in Radiopharmaka des Handels haben die Komplexbildner Ascorbinsäure und Gentisinsäure und die 4-Aminobenzoesäure gefunden.

Den Komplexbildnern haftet allerdings der Nachteil an, dass sie nicht nur mit im Ueberschuss vorhandenen Zinn(II)- und Zinn(IV)ionen Komplexe bilden, sondern ebenso auch mit den $^{99m}$Technetiumionen. Diese Komplexe, weil wasserlöslich, verteilen sich im ganzen Körper und insbesondere in den Weichteilen und sie erzeugen dadurch einen Strahlungsuntergrund, welcher die Bildqualität (Schärfe) der szintigraphischen Darstellungen vermindert. Dies ist eine gemeinsame, unerwünschte Nebenwirkung der Komplexbildner bei ihrer Verwendung als Stabilisatoren.

Hinzu kommt, dass die Organverteilung und die Ausscheidung der aus den $^{99m}$Technetiumionen gebildeten Komplexe manchmal anders als jene der herzustellenden, organ-oder gewebespezifischen Tc-99m-Radiopharmaka sind. Beispielsweise vermindern Cystein oder Ascorbinsäure die Qualität des Knochenagens (Pyrophosphat, Diphosphonate), weil Tc-99m-Cystein sowie Tc-99m-Ascorbinsäure hauptsächlich renal ausgeschieden werden. N-(4-Aminobenzoyl)-glutaminsäure und N-(4-Aminobenzoyl)-asparaginsäure in hoher Konzentration als Stabilisatoren erhöhen die renale Ausscheidung des Knochenagens 2,3-Dicarboxypropan-1,1-diphosphonsäure.

Unerwarteterweise wurde nun gefunden, dass Verbindungen der Formel:

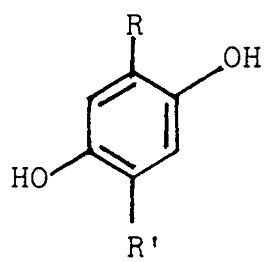

(I)

in welcher R Wasserstoff und R' eine Sulfo- oder Carboxymethylgruppe bedeuten oder R und R' gleiche Bedeutung haben und je eine Sulfo- oder eine Carboxymethylgruppe bedeuten, und ihre wasserlöslichen Salze, welche zwar keine Komplexbildner sind, eine hervorragende stabilisierende Wirkung auf das $^{99m}$Tc-Pertechnetat und die daraus hergestellten Injektionslösungen ausüben. Dieser Befund war umso überraschender, als praktisch alle bisher als Stabilisatoren vorgeschlagene oder verwendete Verbindungen Komplexbildner sind.

Die obige Formel umfasst namentlich die 2,5-Dihydroxybenzolsulfonsäure, die 2,5-Dihydroxybenzol-1,4-disulfonsäure, die 2,5-Dihydroxyphenylessigsäure und die 2,5-Dihydroxy-4-carboxymethyl-phenylessigsäure. Als wasserlösliche Salze kommen vor allem die Alkalimetall- und Ammoniumsalze in Frage; bevorzugt werden die Natriumsalze.

Im physiologisch verträglichen pH-Bereich von etwa 4 bis 9 bilden diese Verbindungen bzw. ihre wasserlöslichen Salze tatsächlich keine beständigen bzw. wasserlöslichen Komplexe, weder mit Uebergangsmetallen, noch mit Schwermetallen, wie aus folgendem Versuch hervorgeht.

Die zu prüfende Verbindung wird in Wasser gelöst, die Lösung wird mit verdünnter Salzsäure auf einen pH-Wert unterhalb 2 eingestellt und mit einer Lösung von $SnF_2$ oder $SnCl_2 \cdot 2H_2O$ in 0,1n-Salzsäure vermischt. Die erhaltene Lösung ist vollkommen klar; bekanntlich ergeben die Zinn(II)ionen nur in stark saurem oder stark alkalischem Medium (im zweiten Fall Bildung von Stannitionen) beständige Lösungen. Hierauf wird das pH durch langsame Zugabe von verdünnter Natronlauge auf einen mittleren Wert erhöht. Bleibt die Lösung klar, so hat die Verbindung die Zinn(II)ionen zu einem Komplex gebunden; entsteht hingegen eine Trübung (basische Zinnsalze, Zinnhydroxid), hat sich kein Komplex gebildet.

Tabelle 2

| pH-Wert und Aussehen wässriger Lösungen von Zinn(II)salzen Inhalt in | | |
|---|---|---|
| 10 ml Lösung | pH-Wert | Aussehen |
| 5 mg $SnCl_2 \cdot 2\ H_2O$ | < 2<br>2 bis 12<br>>12 | klar<br>trüb<br>klar |
| 4 mg $SnF_2$<br>50 mg Ascorbinsäure | 4,7 | klar |
| 5 mg $SnCl_2 \cdot 2\ H_2O$<br>100 mg 1,2-Dihydroxybenzol (Brenzkatechin) | 7,6 | klar |
| 5 mg $SnCl_2 \cdot 2\ H_2O$<br>100 mg 1,2,3-Trihydroxybenzol (Pyrogallol) | 6,0 | klar |
| 4 mg $SnF_2$<br>50 mg 2,5-Dihydroxybenzoesäure (Gentisinsäure) | 6,5 | klar |
| 5 mg $SnCl_2 \cdot 2\ H_2O$<br>100 mg 1,2-Dihydroxybenzol-3,5-disulfonsäure (Tiron) | 6,0 | klar |
| 5 mg $SnCl_2 \cdot 2\ H_2O$<br>100 mg 2,5-Dihydroxybenzolsulfonsäure | 3,0 | trüb |
| 4 mg $SnF_2$<br>50 mg 2,5-Dihydroxybenzol-1,4-disulfonsäure | 3,5 | trüb |
| 5 mg $SnCl_2 \cdot 2\ H_2O$<br>100 mg 2,5-Dihydroxyphenylessigsäure | 2,6 | trüb |
| 5 mg $SnCl_2 \cdot 2\ H_2O$<br>100 mg 2,5-Dihydroxy-4-carboxymethylphenylessigsäure | 4,3 | trüb |

Aus der Tabelle 2 sieht man, dass die Zinn(II)ionen mit Komplexbildnern wie Ascorbinsäure, Gentisinsäure usw. beständige Komplexe bilden, die in schwach saurem bis schwach alkalischem Medium löslich sind.

Nun sind die Verbindungen der Formel (I) im pH-Bereich von 2 bis 9 gut wasserlöslich. Die in diesem pH-Bereich festgestellte Trübung lässt sich also nur dadurch erklären, dass diese Verbindungen mit den Zinn(II)ionen keine Komplexe bilden; sie stehen dadurch in scharfem Gegensatz zu den üblichen Stabilisatoren.

Nichts desto weniger stabilisiert der Zusatz der erwähnten Verbindungen in geringer Konzentration zu Tc-99m-Radiodiagnostika diese gegen die oxidierende Wirkung von im Lauf der Zeit entstehenden Peroxiden und freien Radikalen. Auch nach der Markierung mit hoher Tc-99m-Aktivität (mindestens 5,5 GBq, 150 mCi) erweisen sich solche Lösungen mehrere Stunden - mindestens 4 Stunden - beständig.

Diese Wirkung wird in Tabelle 3 am Beispiel des knochenspezifischen Trägerstoffes Methylendiphosphonsäure (MDP) veranschaulicht. Von dieser Verbindung werden jeweils 20 mg in 1,0 ml Wasser gelöst und in Ampullen vorgelegt; der Ampulleninhalt wird dann mit Zinn(II)fluorid und Natriumpertechnetat und gegebenenfalls mit einem Stabilisator in schwacher Konzentration versetzt. Nach 10 Minuten und 5 bzw. 8 Stunden wird der Gehalt an freiem $^{99m}$Tc-Pertechnetat bestimmt und in % der zur Markierung verwendeten Aktivitätsmenge berechnet. Die Bestimmung erfolgt durch Hochspannungsradioelektrophorese (HVE) auf Whatmanpapier 1 mit Acetatpuffer (0,04 Mol; pH 6,5; 2000 Volt, 30 Minuten) oder durch Dünnschichtradiochromatographie (DRC) auf Whatmanpapier 31 ET mit Aceton als Laufmittel.

Tabelle 3

Stabilisierende Wirkung der Verbindungen (I) auf $^{99m}Tc$-Methylendiphosphonat

| Inhalt der Ampulle (nebst 20 mg MDP in 1,0 ml Wasser) | Aktivitätsmenge zur Markierung | %-Gehalt an freiem Pertechnetat Bestimmung durch HVE 10 Min. | 5 Std. | 8 Std. |
|---|---|---|---|---|
| Vergleichspräparat I<br>0,8 mg Zinn(II)fluorid<br>ohne Stabilisator | 5,4-6,1 GBq (146-165 mCi) | 0 | 14,3 | - 1) |
| Beispiel 1<br>0,8 mg Zinn(II)fluorid<br>0,1 mg Verbindung A,2) Dikaliumsalz | " | 0 | 0 | - |
| Beispiel 2<br>0,8 mg Zinn(II)fluorid<br>0,1 mg 2,5-Dihydroxyphenylessigsäure | " | 0 | 3,8 | - |
| Beispiel 3<br>0,8 mg Zinn(II)fluorid)<br>0,2 mg Verbindung A, Dikaliumsalz | a) 6,8-7,9 GBq (185-210 mCi) | 0 | 0 | - |
| | b)10,7-11,4 GBq (290-310 mCi) | 0 | 0 | 4,5 |
| | c) " | 0 | 0 | 0 |
| Vergleichspräparat II<br>1,2 mg Zinn/fluorid (50% mehr an Reduktionsmittel)<br>ohne Stabilisator | a) 5,4-6,1 GBq (146-165 mCi) | 0 | 8-10 | - |
| | b)10,7-11,4 GBq (290-310 mCi) | 0 | 19-20 | 30-31 |

1): nicht bestimmt

2): 2,5-Dihydroxybenzol-1,4-disulfonsäure

Man sieht, dass in Abwesenheit eines Stabilisators in der Lösung freies Pertechnetat bereits nach 5 Stunden festzustellen ist (14,3 %) und dass selbst die Erhöhung des Gehalts an Zinn(II)ionen die Lösung nicht zu stabilisieren vermag (siehe Vergleichspräparat I bzw. Vergleichspräparat II).

Die zur Stabilisierung ausreichende Konzentration liegt im allgemeinen bei etwa 0,1 bis 6 µmol der Verbindungen der Formel (I); bevorzugt wird meistens eine Konzentration von 0,5 bis 4 µmol.

In der erwähnten extrem niedrigen Konzentration erweisen sich diese Verbindungen bezüglich ihrer stabilisierenden Wirkung den bisher verwendeten Komplexbildnern sogar deutlich überlegen, wie in Tabelle 4 aus dem Gehalt an freiem Pertechnetat 8 Stunden nach der Markierung hervorgeht. Die zur Markierung eingesetzte Aktivitätsmenge betrug jeweils 10,7 - 11,1 GBq (290-300 mCi), die Menge an Methylendiphosphonsäure jeweils 20 mg, jene an Zinn(II)fluorid jeweils 0,8 mg.

Tabelle 4

Vergleich der Wirkung verschiedener Stabilisatoren

| Inhalt der Ampulle (nebst 20 mg MDP und 0,8 mg $SnF_2$ in 1 ml Wasser) | %-Gehalt an freiem Pertechnetat Bestimmung durch HVE | | DRC | |
|---|---|---|---|---|
| | 10 Min. | 8 Std. | 10 Min. | 8 Std. |
| **Beispiel 3** | | | | |
| 0,6 µmol Verbindung A,[1) ] Dikaliumsalz | 0 | 0-2 | 0 | 0-1 |
| **Vergleichspräparat** | | | | |
| III<br>0,6 µmol 4-Amino-benzoesäure | 0 | 2-9 | 0 | 0-2 |
| IV<br>3,6 µmol 4-Hydroxy-benzoesäure | 0 | 6-15 | 0 | 3-5 |
| V<br>3,6 µmol 2,5-Dihydroxy-benzoesäure (Gentisinsäure) | 0 | 14-15 | 0 | 9-12 |

1): 2,5-Dihydroxybenzol-1,4-disulfonsäure

Es lassen sich inbezug auf die Stabilität der Injektionslösungen ähnliche Ergebnisse erhalten, wenn an Stelle der Methylendiphosphonsäure andere Verbindungen als organspezifischer bzw. gewebespezifischer

Trägerstoff eingesetzt werden. Als Beispiele davon seien erwähnt für die Knochenszintigraphie wasserlösliche Phosphate, Polyphosphate und Pyrophosphate und vor allem organische Phosphonsäurederivate wie jene, die u.a. in US 4 229 427 angeführt werden; für die statische und funktionelle Nierenuntersuchung z.B. die Diethylentriaminpentaessigsäure, die Dimercaptobernsteinsäure, ein Glucoheptonat, als hepatobiliäre Trägerstoffe solche der Nitrilotriessigsäuremonoanilid-Reihe wie das Mono-(2,6--dimethyl-, 2,4,6-trimethyl, 2,6-diethyl-, 2,6-diisopropyl- oder 4-butyl)-anilid und ihre Halogenderivate (HIDA-Derivate); ferner partikuläre Präparate, z.B. Albumin-Makroaggregate oder-Mikrosphären; für die Szintigraphie des reticuloendothelialen Systems kolloidale Präparate, z.B. die Mikro-und Nanokolloide aus humanem Serumalbumin oder aus Schwefel; Präparate aus Proteinen, z.B. humanes Serumalbumin oder Fibrinogen; Präparate aus makrocyclischen Verbindungen, z.B. Derivate von Mercaptoacetylglycylglycylglycin ($MAG_3$) und ähnlicher Tripeptide.

Es soll noch hervorgehoben werden, dass die beschriebene stabilisierende Wirkung nicht etwa auf Kosten der Organ- oder Gewebespezifität erreicht wird; diese wird nämlich in vollem Mass gewährleistet, wie aus der Tabelle 5 am Beispiel des $^{99m}$Tc-Methylendiphosphonats (MDP) hervorgeht. Die strikte Beibehaltung der für den-jeweiligen Trägerstoff spezifischen Organ- bzw. Gewebeverteilung in den erfindungsgemäss stabilisierten Injektionslösungen von Radiodiagnostika ist selbstverständlich eine primäre Bedingung für deren praktische Anwendung.

Um die Verteilung der Radioaktivität auf die einzelnen Organe und Gewebe zu bestimmen, werden die Injektionslösungen jeweils Gruppen von 5 Ratten intravenös verabreicht, die Versuchstiere nach 2 Stunden getötet und seziert, die isolierten Organe bzw. Gewebe nach ihrer Art zusammengelegt und davon die Radioaktivität gemessen. Sie wird für die einzelnen Organe und Gewebe in % der insgesamt wiedergefunden Aktivität ± Standardabweichung ausgedrückt.

Tabelle 5

Organverteilung des $^{99m}$Tc-Methylendiphosphonats mit bzw. ohne Stabilisator 1)

| Zusammensetzung der Lösung (1,0 ml) | 20<br>0,8 mg $SnF_2$<br>ohne Stabilisator | 20 mg MDP<br>0,8 mg $SnF_2$<br>0,2 mg 2,5-Dihydroxy-benzol-1,4-di-sulfonsäure<br>(Beispiel 3) | 20 mg MDP<br>0,8 mg $SnF_2$<br>0,2 mg 2,5-Dihydroxy-phenylessigsäure |
|---|---|---|---|
| Blut | 0,40 ± 0,05 | 0,44 ± 0,08 | 0,46 ± 0,18 |
| Lunge | 0,05 ± 0,01 | 0,06 ± 0,01 | 0,05 ± 0,01 |
| Leber | 0,22 ± 0,05 | 0,23 ± 0,05 | 0,15 ± 0,04 |
| Milz | 0,01 ± 0,00 | 0,01 ± 0,00 | 0,01 ± 0,00 |
| Nieren | 0,77 ± 0,15 | 1,11 ± 0,60 | 0,60 ± 0,07 |
| Magen | 0,05 ± 0,01 | 0,06 ± 0,00 | 0,04 ± 0,00 |
| Darmtrakt | 0,31 ± 0,06 | 0,53 ± 0,18 | 0,34 ± 0,07 |
| Knochen | 52,31 ± 2,04 | 57,25 ± 2,50 | 52,24 ± 1,92 |
| Muskel | 0,90 ± 0,16 | 1,15 ± 0,39 | 0,73 ± 0,18 |
| Schilddrüse | 0,00 ± 0,00 | 0,00 ± 0,00 | 0,00 ± 0,00 |
| Urin und Blase | 44,97 ± 2,05 | 39,18 ± 3,28 | 42,88 ± 0,58 |
| Summe | 99,99 ± 2,90 | 100,02 ± 4,19 | 97,50 ± 2,09 |

1): in % der wiedergefundenen Aktivität

Bei umfassendem Vergleich mit den zur Stabilisierung von $^{99m}$Tc-markierten Präparaten bisher vorgeschlagenen oder verwendeten Verbindungen zeichen sich die Verbindungen der Formel I durch eine höhere oder gleich ausgeprägte Wirksamkeit bei sehr niedriger Konzentration aus und sie ermöglichen andererseits die Erstellung von szintigraphischen Darstellungen, deren Qualität nicht durch einen Strahlungsuntergrund vermindert ist.

Gemäss der Erfindung werden also stabile, gebrauchsfertige, $^{99m}$Tc-markierte Präparate zur Verfügung gestellt, welche zur Anwendung als Radiodiagnostika bestimmt sind und (a) eine als organ- bzw. gewebespezifischer Trägerstoff fungierende Substanz, die mit dem Radionuklid Tc-99m markiert ist, und (b) eine

Verbindung der Formel (I) oder ein wasserlösliches Salz derselben in einer zur Stabilisierung der Präparate gegen oxidierende Einflüsse ausreichenden Menge enthalten.

Die Herstellung der erfindungsgemässen Präparate erfolgt dadurch, dass man der als Trägerstoff vorgesehenen oder fungierenden Substanz (a) vor, während oder nach deren Markierung mit dem Radionuklid Tc-99m die oben mit (b) bezeichnete Verbindung als Stabilisator in einer gegenüber der Konzentration an Trägerstoff untergeordneten Konzentration zusetzt.

Die Markierung selbst erfolgt durch Zugabe eines Pertechnetats und Einwirkung eines Reduktionsmittels, wie eines Zinn(II)salzes, eines Eisen(II)salzes, eines Chrom(II)salzes oder von Natriumborhydrid oder Natriumdithionit; vorzugsweise wird Zinn(II)chlorid oder Zinn(II)fluorid als Reduktionsmittel verwendet.

Im folgenden werden die drei Verfahrensvarianten und bevorzugte Ausführungsformen der Erfindung ausführlicher beschrieben.

I. Zugabe des Stabilisators vor der Markierung

Die als Stabilisator ausgewählte Verbindung wird einer Lösung des Trägerstoffes und des Reduktionsmittels zugegeben; vorzugsweise wird die Lösung der drei Komponenten hierauf lyophilisiert. Das Lyophilisat enthält also den organspezifischen oder gewebespezifischen Trägerstoff, das Reduktionsmittel und die als Stabilisator vorgesehene Verbindung; es stellt ein bevorzugtes Mittel zur Ausführung des oben definierten Verfahrens dar und wird mit Vorteil unter Vakuum in einem Durchstechfläschchen dargeboten.

Im Latoratorium oder in der Klinik genügt es dann, das Lyophilisat mit der berechneten Menge Tc-99m-Eluat aus dem Generator zu versetzen, um das gebrauchsfertige, über mehrere Stunden stabile Radiodiagnostikum zu erhalten.

II. Zugabe des Stabilisators während der Markierung

Gemäss der zweiten Verfahrensvariante liegt die als Stabilisator vorgesehene Verbindung im Eluat vor, welches das Tc-99m-Pertechnetat enthält und mit welchem die Markierung des Trägerstoffes erfolgen soll. Eine vorteilhafte Ausführungsform der Erfindung besteht darin, dass die erwähnte Verbindung in fester Form und unter Vakuum in einem zur Aufnahme des Eluates aus dem Tc-99m-Generator bestimmten Durchstechfläschchen dargeboten wird, das Eluat in das Durchstechfläschchen eingeführt wird und die Lösung von Eluat und Stabilisator zur Markierung des Trägerstoffes unter Mitwirkung eines Reduktionsmittels verwendet wird.

Man kann aber auch den Stabilisator in den Tc-99m-Generator selbst, vor der Entnahme des radioaktives Pertechnetat enthaltenden Eluates, einfügen und auf diese Weise ein Eluat erhalten, welches den Stabilisator bereits enthält. Bei dieser Ausführungsform wird der Stabilisator dem Trägerstoff ebenfalls während der Markierung zugegeben.

III. Zugabe des Stabilisators nach der Markierung

Die dritte Verfahrensvariante besteht darin, dass man die als Stabilisator ausgewählte Verbindung dem mit Tc-99m bereits markierten Präparat zusetzt, was durch Versetzen mit einer wässrigen Lösung oder einem Lyophilisat der erwähnten Verbindung geschieht.

Beispiel 1

1,0 g Methylendiphosphonsäure (MDP) wird in 25 ml destilliertem Wasser vorgelegt. Der pH der sauren Lösung wird mit 2n-NaOH auf 7,0 eingestellt. Nachfolgend werden 4,0 ml 1%ige Zinn(II)fluorid-Lösung in 0,1n-HCl und 1,0 ml einer 0,5%igen Lösung des 2,5-Dihydroxybenzol-1,4-disulfonsäure-dikaliumsalzes in destilliertem Wasser unter Rühren zugegeben. Die so hergestellte Lösung wird mit 1n-HCl bzw. 1n-NaOH auf pH 6,5 eingestellt und mit destilliertem Wasser auf ein Endvolumen von 50 ml verdünnt. Nach der Sterilfiltration über 0,2 $\mu$ Sterilfilter wird das Filtrat in Portionen von 1,0 ml in Fläschchen lyophilisiert.

| | |
|---|---|
| Fläschcheninhalt: | 20 mg MDP |
| | 0,8 mg Zinn(II)fluorid |
| | 0,1 mg 2,5-Dihydroxybenzol-1,4-disulfonsäure-dikaliumsalz |
| Analysenbefund : | siehe Tabelle 3 |

Beispiel 2

Wie im Beispiel 1 wird eine Injektionslösung der folgenden Zusammensetzung hergestellt:

| | |
|---|---|
| Fläschcheninhalt: | 20 mg MDP |
| | 0,8 mg Zinn(II)fluorid |
| | 0,1 mg 2,5-Dihydroxyphenylessigsäure |
| Analysenbefund : | siehe Tabelle 3 |

Beispiel 3

2,0 g Methylendiphosphonsäure (MDP) werden in 80 ml destilliertem Wasser vorgelegt. In die entstehende saure Lösung (pH 1,8) werden 80 mg festes Zinn(II)fluorid gelöst. Danach werden 21 mg festes 2,5-Dihydroxy-1,4-benzoldisulfonsäure-dikaliumsalz der Lösung zugegeben und unter Rühren gelöst. Nach pH-Einstellung auf 7,0 mit 2n-Natronlauge wird die Lösung mit destilliertem Wasser auf ein Endvolumen von 100 ml verdünnt und anschliessend durch einen 0,2 µ Sterilfilter filtriert. Das Filtrat wird in Portionen von 1 ml in Fläschchen lyophilisiert.

| | |
|---|---|
| Fläschcheninhalt: | 20 mg MDP |
| | 0,8 mg $SnF_2$ |
| | 0,2 mg (0,6 µmol) 2,5-Dihydroxybenzol-1,4-disulfonsäuredikaliumsalz |
| Analysenbefund : | siehe Tabelle 3 |

Vergleichspräparate I und II

Zur Gegenüberstellung mit den Beispielen 1 bis 3 werden gemäss Beispiel 1 Präparate ohne Zusatz eines Stabilisators hergestellt und im folgenden als Vergleichspräparat I bzw. II bezeichnet.

| | | I | II |
|---|---|---|---|
| Fläschcheninhalt: | MDP<br>$SnF_2$ | 20 mg<br>0,8 mg | 20 mg<br>1,2 mg |

Vergleichspräparate III, IV und V

Zur Gegenüberstellung mit dem Beispiel 3 werden gemäss Beispiel 1 Präparate mit den bekannten Stabilisatoren hergestellt. Im folgenden wird der Fläschcheninhalt (in 1,0 ml) angegeben; der Analysenbefund befindet sich in der Tabelle 4.

| III | IV | V |
|---|---|---|
| 20 mg MDP<br>0,8 mg $SnF_2$<br>0,08 mg 4-Aminobenzoesäure (0,6 µmol) | 20 mg MDP<br>0,8 mg $SnF_2$<br>0,5 mg 4-Hydroxybenzoesäure (3,6 µmol) | 20 mg MDP<br>0,8 mg $SnF_2$<br>0,55 mg 2,5-Dihydroxybenzoesäure (Gentisinsäure) (3,6 µmol) |

Beispiel 4

Zu 0,2-ml einer 0,1%igen Lösung des Dikaliumsalzes der 2,5-Dihydroxybenzol-1,4-disulfonsäure (Verbindung A) in Wasser werden 5 ml frisch eluiertes $^{99m}$Tc-Eluat der Aktivität 17,17 GBq (464 mCi) zugesetzt.

Der Gehalt an Peroxid im so hergestellten $^{99m}$Tc-Eluat wird mittels Merckoquant-Teststäbchen für Peroxidtests (Merck Art. Nr. 10011) semiquantitativ bestimmt. Die Bestimmungen erfolgen bei verschiedenen Standzeiten nach der Behandlung des Tc-99m-Eluats mit der erwähnten Disulfonsäure.

| Standzeit | 10 Min. | 5 Std. | 8 Std. |
|---|---|---|---|
| Peroxidgehalt | 0 ppm | 0 ppm | 0 ppm |

Beispiel 5

Das mit dem Dikaliumsalz der 2,5-Dihydroxybenzol-1,4-disulfonsäure (Verbindung A) behandelte $^{99m}$Tc-Eluat aus dem Beispiel 4 wird für die Markierung des Vergleichspräparates I - bestehend aus 20 mg MDP und 0,8 mg Zinn(II)fluorid in 1,0 ml - verwendet. Der Gehalt an freiem Pertechnetat in den markierten MDP-Lösungen wird durch Dünnschichtsradiochromatographie auf Whatmanpapier 31 ET mit Aceton als Laufmittel bestimmt und in % der zur Markierung eingesetzten Aktivität ausgedrückt. Die Ergebnisse finden sich in der Tabelle 6.

Tabelle 6

Gehalt an freiem Pertechnetat in % der Markierungsaktivität

| Stabilisator | 0,064 mg Verbindung A | 0,128 mg Verbindung A |
|---|---|---|
| Aktivität des $^{99m}$Tc-Eluates | 5,437 GBq (147 mCi) | 10,7 GBq (289 mCi) |
| Standzeit nach der Markierung | | |
| 10 Min. | 0% | 0% |
| 5 Std. | 0% | 0% |
| 8 Std. | 6,6% | 2,4% |

Beispiel 6

2,0 g Methylendiphosphonsäure (MDP) werden in 80 ml destilliertem Wasser gelöst. Unter Stickstoffatmosphäre werden 80 mg festes Zinn(II)fluorid in die MDP-Lösung zugegeben. Nach pH-Einstellung auf 5,0 durch 2n-NaOH werden 82 mg 2,5-Dihydroxybenzolsulfonsäure darin gelöst. Durch weitere Zugabe von 2n-NaOH wird die Lösung auf ein pH von 7,0 eingestellt und mit destilliertem Wasser auf ein Endvolumen von 100 ml verdünnt. Die Lösung wird durch einen 0,2 $\mu$ Sterilfilter filtriert und das Filtrat wird in Portionen von 1 ml in Fläschchen lyophilisiert.

Fläschcheninhalt: 20,0 mg MDP

| | |
|---|---|
| | 0,8 mg Zinn(II)fluorid |
| | 0,82 mg (3,6 µmol) 2,5-Dihydroxybenzolsulfonsäure |
| Analysenbefund : | Radiochromatographie : siehe Tabelle 7 |
| | Organverteilung : siehe Tabelle 8 |

Beispiel 7

Wie im Beispiel 6 wird ein Präparat der nachstehenden Zusammensetzung hergestellt.

| | |
|---|---|
| Fläschcheninhalt: | 20,0 mg MDP |
| | 0,8 mg Zinn(II)fluorid |
| | 0,6 mg (3,6 µmol) 2,5-Dihydroxyphenylessigsäure |
| Analysenbefund : | Radiochromatographie : siehe Tabelle 7 |
| | Organverteilung : siehe Tabelle 8 |

Beispiel 8

Wie im Beispiel 6 wird ein Präparat der nachstehenden Zusammensetzung hergestellt.

| | |
|---|---|
| Fläschcheninhalt: | 20,0 mg MDP |
| | 0,8 mg Zinn(II)fluorid |
| | 0,81 mg (3,6 µmol) 2,5-Dihydroxy-4-carboxymethylphenylessigsäure |
| Analysenbefund : | Radiochromatographie : siehe Tabelle 7 |
| | Organverteilung : siehe Tabelle 8 |

## Tabelle 7

Gehalt an freiem Pertechnetat in % der Markierungsaktivität

| | Beispiel 6 | Beispiel 7 | Beispiel 8 |
|---|---|---|---|
| Markierungs-aktivität (per Ampulle) | 12'000 MBq (324 mCi) | 11'000 MBq (297 mCi) | 11'000 MBq (297 mCi) |
| HVE-Befund 1) | | | |
| 10 Min. | 0 | 0 | 0-1 |
| 5 Std. | 0 | 0 | - |
| 8 Std. | 0-1 | 0-1 | 0-1 |
| RC-Befund 2) | | | |
| 10 Min. | 0 | 0 | 0 |
| 5 Std. | 0 | 0 | - |
| 8 Std. | 0-1 | 0-1 | 0-1 |

1): Durch Hochspannungsradioelektrophorese auf Whatmanpapier 1 in Acetatpuffer (0,04 mol; pH 6,5; 30 Min. bei 2'000 V).

2): Durch Radiochromatographie auf Gelman ITLC-SG in Aceton.

Tabelle 8

| Organverteilung des $^{99m}$Tc-Methykendiphosphonats[1)] (Ratten, n = 5, 2 Std. nach iv-Verabreichung) | | | |
|---|---|---|---|
| Markierungsaktivität (per Ampulle) | Beispiel 6 | Beispiel 7 | Beispiel 8 |
| | 12'000 MBq (324 mCi) | 11'000 MBq (297 mCi) | 11'000 MBq (297 mCi) |
| Blut | 0,47 ± 0,16 | 0,58 ± 0,06 | 0,62 ± 0,22 |
| Lunge | 0,06 ± 0,01 | 0,05 ± 0>03 | 0,08 ± 0,03 |
| Leber | 0,21 ± 0,07 | 0,29 ± 0,05 | 0,27 ± 0,08 |
| Milz | 0,02 ± 0,00 | 0,02 ± 0,00 | 0,02 ± 0,00 |
| Nieren | 0,68 ± 0,14 | 1,31 ± 0,34 | 0,81 ± 0,41 |
| Magen | 0,08 ± 0,03 | 0,11 ± 0,02 | 0,25 ± 0,04 |
| Darmtrakt | 0,45 ± 0,09 | 0,43 ± 0,05 | 0,51 ± 0,29 |
| Knochen | 41,80 ± 4,91 | 53,54 ± 7,95 | 46,07 ± 0,48 |
| Muskel | 0,96 ± 0,25 | 1,16 ± 0,13 | 1,00 ± 0,29 |
| Schilddrüse | 0,00 ± 0,00 | 0,00 ± 0,00 | 0,01 ± 0,00 |
| Urin | 54,18 ± 7,64 | 38,67 ± 4,83 | 45,37 ± 1,32 |
| Summe | 98,91 | 96,16 | 95,01 |

1):Mittelwert in % der eingesetzten Markierungsaktivität

**Patentansprüche**

1. Stabiles, gebrauchsfertiges, mit dem Radionuklid Tc-99m markiertes Präparat zur Anwendung als Radiodiagnostikum, dadurch gekennzeichnet, dass es eine als organspezifischer oder gewebespezifischer Trägerstoff fungierende Substanz, welche mit dem Radionuklid Tc-99m markiert ist, und zur Stabilisierung des Präparates gegen oxidierende Einflüsse eine ausreichende Menge einer Verbindung der Formel:

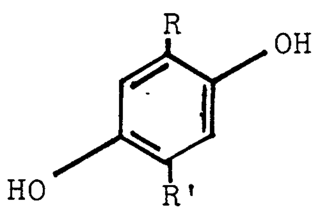

(I)

in welcher R Wasserstoff und R' eine Sulfo- oder Carboxymethylgruppe bedeuten oder R und R' gleiche Bedeutung haben und je eine Sulfo- oder eine Carboxymethylgruppe bedeuten, oder eines wasserlösliches Salzes derselben in einer untergeordneten Konzentration gegen-über jener an Trägerstoff enthält.

2. Stabiles Präparat nach Anspruch 1, dadurch gekennzeichnet, dass als wasserlösliches Salz ein Alkalimetall- oder ein Ammmoniumsalz, vorzugsweise ein Natriumsalz, verwendet wird.

3. Stabiles Präparat nach Anspruch 1 oder 2, dadurch gekennzeichnet, dass als Verbindung der Formel I die 2,5-Dihydroxybenzol-1,4-disulfonsäure oder ihr Natriumsalz verwendet wird.

4. Stabiles Präparat nach Anspruch 1, dadurch gekennzeichnet, dass die zur Stabilisierung verwendete Verbindung in einer Konzentration von 0,1 bis 6,0 µmol, vorzugsweise von 0,5 bis 4,0 µmol je 10,7-11,1 GBq zur Markierung eingesetzte Aktivitätsmenge, vorliegt.

5. Stabiles Präparat nach Anspruch 1, dadurch gekennzeichnet, dass der Trägerstoff eine für die Knochenszintigraphie geeignete Substanz, insbesondere ein Pyrophosphat oder ein Diphosphonsäurederivat wie Methylendiphosphonsäure, Hydroxymethylendiphosphonsäure oder 2,3-Dicarboxypropan-1,1-diphosphonsäure ist.

6. Stabiles Präparat nach Anspruch 1, dadurch gekennzeichnet, dass der Trägerstoff eine für die statische oder funktionelle Nierenuntersuchung geeignete Substanz, insbesondere die Diethylentriaminpentaessigsäure, die Dimercaptobernsteinsäure, ein Glucoheptonat oder ein Derivat des Mercaptoacetylglycylglycylglycins oder ähnlicher Tripeptide ist.

7. Stabiles Präparat nach Anspruch 1, dadurch gekennzeichnet, dass der Trägerstoff eine für die Szintigraphie des hepatobiliären Systems geeignete Substanz, insbesondere ein Derivat der Nitrilotriessigsäure-monoanilid Reihe ist.

8. Stabiles Präparat nach Anspruch 1, dadurch gekennzeichnet, dass der Trägerstoff eine für die Szintigraphie des reticuloendothelialen Systems geeignete Substanz, insbesondere ein kolloidales Präparat wie Mikro- und Nanokolloide aus humanem Serumalbumin oder Schwefel Rhonium ist.

9. Verfahren zur Herstellung des stabilen Präparates nach Anspruch 1, dadurch gekennzeichnet, dass man der als organspezifischer oder gewebespezifischer Trägerstoff vorgesehenen bzw. fungierenden Substanz vor, während oder nach deren Markierung mittels eines Pertechnetats und eines Reduktionsmittels eine Verbindung der Formel (I) oder ein wasserlösliches Salz derselben in einer untergeordneten Konzentration gegenüber jener an Trägerstoff zusetzt.

10. Verfahren nach Anspruch 9, dadurch gekennzeichnet, dass als Reduktionsmittel ein Zinn(II)salz, ein Eisen(II)salz, ein Chrom(II)salz, Natriumborhydrid oder Natriumdithionit, vorzugsweise Zinn(II)chlorid oder Zinn(II)fluorid, verwendet wird.

11. Mittel zur Ausführung des Verfahrens nach Anspruch 9, welches als Zubereitung aus dem Reduktionsmittel und der zur Stabilisierung vorgesehenen Verbindung vorliegt.

12. Mittel zur Ausführung des Verfahrens nach Anspruch 9, welches als Zubereitung aus dem organspezifischen oder gewebespezifischen Trägerstoff, dem Reduktionsmittel und der zur Stabilisierunt vorgesehenen Verbindung vorliegt.

13. Mittel nach Anspruch 12, welches in Form eines Lyophilisats der Zubereitung, vorzugsweise unter Vakuum in einem Durchstechfläschchen, vorliegt.

14. Mittel zur Ausführung des Verfahrens nach Anspruch 9, bestehend aus der zur Stabilisierung vorgesehenen Verbindung in fester Form und unter Vakuum in einem zur Aufnahme des Eluats aus dem $^{99m}$Tc-Generator bestimmten Durchstechfläschchen.

15. Mittel zur Ausführung des Verfahrens nach Anspruch 9, welches als Zubereitung aus der zur Stabilisierung vorgesehenen Verbindung und einem Eluat aus dem $^{99m}$Tc-Generator vorliegt.

16. Mittel zur Ausführung des Verfahrens nach Anspruch 9, bestehend aus der zur Stabilisierung vorgesehenen Verbindung in Form einer wässrigen Lösung oder eines Lyophilisats, mit welcher bzw. welchem das radioaktiv markierte Präparat versetzt werden soll.

## Claims

1. A stable preparation ready for use and labelled with the radionuclide Tc-99m for use as a radio diagnostic agent, characterised in that it contains a substance acting as an organ-specific or tissue-specific carrier substance labelled with the radionuclide Tc-99m, and, to stabilise the preparation against oxidising influences, an adequate quantity of a a compound of the formula:

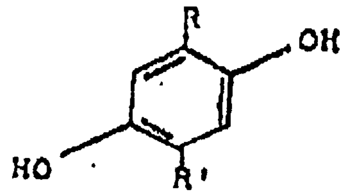

(I)

where R denotes hydrogen and R' denotes a sulphomethyl group or carboxymethyl group or R and R' have the same meaning and each denotes a sulphomethyl group or a carboxymethyl group, or a water-soluble salt thereof in a minor concentration in comparison with that of the carrier substance.

**2.** A stable preparation according to claim 1, characterised in that the water-soluble salt used is an alkali metal or an ammonium salt, preferably a sodium salt.

**3.** A stable preparation according to claim 1 or 2, characterised in that the formula I compound used is 2,5-dihydroxybenzene-1,4-disulphonic acid or its sodium salt.

**4.** A stable preparation according to claim 1, characterised in that the compound used for stabilisation is present in a concentration of from 0.1 to 6.0 µmol, preferably from 0.5 to 4.0 µmol par 10.7 - 11.1 GBq of the quantity of activity used far labelling.

**5.** A stable preparation according to claim 1, characterised in that the carrier substance is a substance suitable for bone scintigraphy, more particularly a pyrophosphate or a diphosphonic acid derivative, such as methylene diphosphonic acid, hydroxymethylene diphosphonic acid or 2,3-dicarboxypropane-1,1-diphosphonic acid.

**6.** A stable preparation according to claim 1, characterised in that the carrier substance is a substance suitable for statistical or functional kidney examination, more particularly diethylene triamine pentaacetic acid, dimercaptosuccinic acid, a glucoheptonate or a derivative of mercaptoacetylglycyl glycylglycin or similar tripeptides.

**7.** A stable preparation according to claim 1, characterised in that the carrier substance is a substance suitable for the scintigraphy of the hepatobiliary system, more particularly a derivative of the nitrilotriacetic acid monoanilide series.

**8.** A stable preparation according to claim 1, characterised in, that the carrier substance is a substance suitable for the scintigraphy of the reticuloendothelial system, more particularly a colloidal preparation such as microcolloids and nanocolloids of human serum albumin or sulphur.

**9.** A process for the preparation of the stable preparation according to claim 1, characterised in that there is added to the substance functioning or provided as an organ-specific or tissue-specific carrier substance, either before, during or after its labelling by means of a pertechnetate and a reducing agent, a compound of formula (I) or a water-soluble salt thereof in a minor concentration in comparison with that of the carrier substance.

**10.** A process according to claim 9, characterised in that the reducing agent used is a tin (II) salt, an iron (II) salt, a chromium (II) salt, sodium borohydride or sodium dithionite, preferably tin (II) chloride or tin (II) fluoride.

**11.** An agent for performing the method according to claim 9, which is in the form of a preparation made from the reducing agent and from the compound provided for stabilisation.

**12.** An agent for performing the method according to claim 9, which is present in the form of a preparation made from the organ-specific or tissue-specific carrier substance, the reducing agent, and the compound provided for stabilisation.

13. An agent according to claim 12, which is in the form of a lyophilisate of the preparation, preferably in a vacuum in a pierceable flask.

14. An agent for performing the method according to claim 9, consisting of the stabilising compound in solid form and in a vacuum in a pierceable flask intended to receive the eluate from the $^{99m}$Tc generator.

15. An agent for performing the method according to claim 9, which is present in the form of a preparation made from the stabilising compound and an eluate from the $^{99m}$Tc generator.

16. An agent for performing the method according to claim 9, consisting of the compound provided for stabilisation in the form of an aqueous solution or a lyophilisate, with either of which the radioactively labelled preparation is to be mixed

**Revendications**

1. Composition stable, prête à l'emploi, marquée avec le radionucléide Tc-99 m destinée à être utilisée comme radiodiagnostic, caractérisée en ce que
elle comprend une substance ayant le rôle de vecteur à affinité spécifique pour les organes et les tissus et marquée avec le radionucléide Tc-99 m, ainsi qu'un composé en quantité suffisante pour stabiliser ladite composition contre une attaque oxydante, ledit composé répondant à la formule

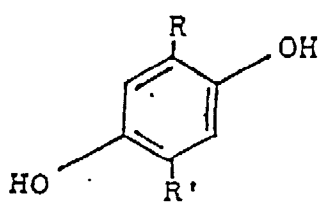

(I)

dans laquelle soit R est l'hydrogène et R' un groupement thio- ou carboxy-méthyle soit R et R' sont identiques et sont un groupement thio- ou carboxy-méthyle, ou bien un sel dudit composé, soluble dans l'eau, en concentration inférieure à celle dudit vecteur.

2. Composition stable selon la revendication 1, caractérisée en ce que ledit sel soluble dans l'eau est un sol de métal alcalin ou d'ammonium et de préférence un sel de sodium.

3. Composition stable selon la revendication 1 ou 2, caractérisée en ce que le composé de formule I est l'acide-dihydroxy-2,5-benzol-disulfonique-1,4 ou son sel de sodium.

4. Composition stable selon la revendication 1, caractérisée en ce que la concentration dudit composé de stabilisation est comprise entre 0,1 et 6,0 $\mu$ moles, et de préférence entre 0,5 et 4,0 $\mu$ moles pour un marquage d'une activité de 10,7 à 11,1 GBq.

5. Composition stable selon la revendication 1, caractérisée en ce que le vecteur est une substance appropriée à la scintigraphie osseuse et en particulier un pyrophosphate ou un dérivé de l'acide diphosphonique tel que l'acide-méthylène-diphosphonique, l'acide-hydroxyméthylène-diphosphonique ou l'acide-dicarboxy-2,3-propane-diphosphonique-1,1.

6. Composition stable selon la revendication 1, caractérisée on ce que le vecteur est une substance appropriée à l'analyse statique ou fonctionnelle des reins et en particulier l'acide diéthyltriamino-pentaacétique, l'acide dimercapto-succinique, un glucoheptonate ou un dérivé du mercaptoacétylglycylglycylglycine ou d'un tripeptide analogue.

7. Composition stable selon la revendication 1, caractérisée en ce que le vecteur est une substance appropriée à la scintigraphie du système hépatobiliaire et en particulier un dérivé de la série dos acides-nitrilotriacétiques-monoanilide.

8. Composition stable selon la revendication 1, caractérisée en ce que le vecteur est une substance appropriée à la scintigraphie du système réticuloendothélial et en particulier une composition colloïdale telle qu'un micro- ou nano-colloïde de sérum'albumine humaine ou de soufre.

9. Procédé pour préparer une composition stable selon la revendication 1, caractérisé en ce qu'on ajoute, à la substance prévue et ayant le rôle de vecteur à affinité spécifique pour les organes et les tissus, un composé répondant à la formule (I) ou un sel de ce dernier, soluble dans l'eau, en quantité inférieure à celle du vecteur, pendant ou après le marquage au moyen d'un pertechnétate et d'un agent réducteur.

10. Procédé selon la revendication 9, caractérisé en ce que l'agent réducteur est un sel stanneux (II), un sel ferreux (II), un sel chromeux (II), le borohydrure de sodium ou' l'hyposulfite de sodium et de préférence le chlorure stanneux (II) ou le fluorure stanneux (II).

11. Milieu pour mettre en oeuvre le procédé selon la revendication 9, se présentant sous la forme d'une préparation comprenant l'agent réducteur et le composé prévu pour la stabilisation.

12. Milieu pour mettre en oeuvre le procédé selon la revendication 9, se présentant sous la forme d'une préparation comprenant le vecteur à affinité spécifique pour les organes et les tissus, l'agent réducteur et le composé prévu pour la stabilisation.

13. Milieu selon la revendication 12, se présentant sous la forme d'une préparation lyophilisée et de préférence dans un flacon sous vide à perforer.

14. Milieu pour mettre en oeuvre le procédé selon la revendication 9, consistant en le composé prévu pour la stabilisation, à l'état solide et en un flacon sous vide à perforer destiné à recevoir le produit d'élution du générateur-$^{99m}$ Tc.

15. Milieu pour mettre en oeuvre le procédé selon la revendication 9, se présentant sous la forme du composé prévu pour la stabilisation et d'un produit d'élution du générateur-$^{99m}$ Tc.

16. Milieu pour mettre en oeuvre le procédé selon la revendication 9, consistant en le composé prévu pour la stabilisation, sous forme d'une solution aqueuse ou sous forme lyophilysée, avec lequel la composition marquée radioactive est ou sera mélangée.